# EUROPEAN PATENT APPLICATION

(11) **EP 4 806 950 A1**
(43) Date of publication of application: **16.09.2026**
(21) Application number: 26164230.0
(22) Date of filing: 12.03.2026
(51) Int. Cl.: C08J 11/16

(54) **DECOMPOSITION METHOD FOR POLYESTER-BASED RESIN AND DECOMPOSITION APPARATUS FOR POLYESTER-BASED RESIN**

(30) Priority: 12.03.2025 JP 2025039652
(71) Applicant: Nitto Denko Corporation, Ibaraki-shi, Osaka 567-8680 (JP)
(72) Inventor: YAMADA, Yosuke, Ibaraki-shi, Osaka, 567-8680 (JP); YOSHIDA, Chihiro, Ibaraki-shi, Osaka, 567-8680 (JP)
(74) Representative: Hoffmann Eitle

(57) **Abstract**

Provided is a decomposition method for a polyester-based resin, which can efficiently produce a diol and a dicarboxylate from the polyester-based resin in a short time period without using a solvent. Also provided is a decomposition apparatus for a polyester-based resin, which decomposes the polyester-based resin to efficiently produce a diol and a dicarboxylate in a short time period without using a solvent. A decomposition method for a polyester-based resin according to an embodiment of the present invention includes kneading a mixture containing a polyester-based resin and a solid alkali compound to produce a diol and a dicarboxylate. A decomposition apparatus for a polyester-based resin according to an embodiment of the present invention is a decomposition apparatus configured to decompose a polyester-based resin to produce a diol and a dicarboxylate, and includes a kneading portion configured to knead a mixture containing the polyester-based resin and a solid alkali compound.

## Description

### BACKGROUND OF THE INVENTION

### 1. Field of the Invention

The present invention relates to a decomposition method for a polyester-based resin and a decomposition apparatus for a polyester-based resin.

### 2. Description of Related Art

A polyester-based resin typified by polyethylene terephthalate (PET) can have high transparency and excellent mechanical characteristics, and hence has been used as a material for many products and members such as a base material used for an optical protective film.

Many of the polyester-based resins such as PET have been produced from raw materials derived from petroleum, which is a depleted resource. Accordingly, from the viewpoint of effective utilization of resources, a recycling technology for the polyester-based resins has been required.

Mechanical recycling (sometimes referred to as "material recycling") has been known as a recycling method for polyester-based resins. The mechanical recycling is a recycling method in which a polyester-based resin is recycled in the state of the resin. However, in the mechanical recycling, there is a problem in that the quality deterioration of the polyester-based resin occurs when the recycling is repeated a plurality of times.

Chemical recycling has been known as a recycling method that solves the above-mentioned problem in the mechanical recycling. The chemical recycling is a recycling method in which a polyester-based resin is depolymerized into a raw material monomer, and the resultant raw material monomer is purified and then repolymerized to recycle the polyester-based resin.

As a method of depolymerizing polyethylene terephthalate into terephthalic acid or a salt thereof and ethylene glycol, there is a report on a method in which polyethylene terephthalate is mixed with a mixture of a halogenated solvent for swelling the polyethylene terephthalate, an alcohol, and a hydroxide over about 1 hour (JP 2021-073332 A). However, in the method, there is a problem in that an environmental load is large because the halogenated solvent needs to be used to swell the polyethylene terephthalate.

As a technology related to the depolymerization of polyethylene terephthalate, there is a report on the following decomposition method for polyethylene terephthalate in a waste plastic mixture (JP 2024-006038 A): a waste plastic mixture containing polyethylene terephthalate and a chlorine-containing polymer is heated in an airtight container to a temperature of 200°C or more and 330°C or less so that a mixture of terephthalic acid, mono(2-chloroethyl) terephthalate, and bis(2-chloroethyl) terephthalate may be separated. However, in the method, there is a problem in that production efficiency is poor because heating for a time period as long as 2 hours or more at a temperature of 200°C or more in the airtight container is required.

### SUMMARY OF THE INVENTION

An object of the present invention is to provide a decomposition method for a polyester-based resin, which can efficiently produce a diol and a dicarboxylate from the polyester-based resin in a short time period without using a solvent. Another object of the present invention is to provide a decomposition apparatus for a polyester-based resin, which decomposes the polyester-based resin to efficiently produce a diol and a dicarboxylate in a short time period without using a solvent.

The inventors of the present invention have conducted investigations on a technology capable of producing a diol and a dicarboxylate from a polyester-based resin in a short time period without using a solvent. As a result, the inventors have found that when a mixture containing a polyester-based resin and a solid alkali compound is kneaded, the depolymerization of the polyester-based resin proceeds without use of a solvent and in a short time period, and hence a diol and a dicarboxylate can be produced therefrom. Thus, the inventors have completed the present invention.
[1] A decomposition method for a polyester-based resin according to an embodiment of the present invention includes kneading a mixture containing a polyester-based resin and a solid alkali compound to produce a diol and a dicarboxylate.
[2] In the decomposition method for a polyester-based resin according to the above-mentioned item [1], a molar equivalent of the solid alkali compound relative to 1 molar equivalent of the polyester-based resin may be 0.8 molar equivalent or more.
[3] In the decomposition method for a polyester-based resin according to the above-mentioned item [2], the molar equivalent of the solid alkali compound relative to 1 molar equivalent of the polyester-based resin may be from 1.0 molar equivalent to 1.5 molar equivalents.
[4] In the decomposition method for a polyester-based resin according to any one of the above-mentioned items [1] to [3], a temperature of the kneading may be 50°C or more and less than 400°C.
[5] In the decomposition method for a polyester-based resin according to the above-mentioned item [4], the temperature of the kneading may be 120°C or more and less than 270°C.
[6] In the decomposition method for a polyester-based resin according to any one of the above-mentioned items [1] to [5], a liquid product containing the diol and a solid product containing the dicarboxylate may be obtained by the kneading.
[7] In the decomposition method for a polyester-based resin according to any one of the above-mentioned items [1] to [6], the dicarboxylate may be subjected to neutralization crystallization to produce a dicarboxylic acid.
[8] A decomposition apparatus for a polyester-based resin according to an embodiment of the present invention is a decomposition apparatus configured to decompose a polyester-based resin to produce a diol and a dicarboxylate, and the decomposition apparatus includes a kneading portion configured to knead a mixture containing the polyester-based resin and a solid alkali compound.
[9] The decomposition apparatus for a polyester-based resin according to the above-mentioned item [8] may further include: a first discharge portion configured to discharge a liquid product containing the diol; and a second discharge portion configured to discharge a solid product containing the dicarboxylate.

The present invention can provide the decomposition method for a polyester-based resin, which can efficiently produce a diol and a dicarboxylate from the polyester-based resin in a short time period without using a solvent. The present invention can also provide the decomposition apparatus for a polyester-based resin, which decomposes the polyester-based resin to efficiently produce a diol and a dicarboxylate in a short time period without using a solvent.

### DETAILED DESCRIPTION OF THE INVENTION

When the expression "weight" is used herein, the expression may be replaced with "mass" that has been commonly used as an SI unit that represents a weight.

A decomposition method for a polyester-based resin according to an embodiment of the present invention includes kneading a mixture containing a polyester-based resin and a solid alkali compound to produce a diol and a dicarboxylate.

In the decomposition method for a polyester-based resin according to the embodiment of the present invention, a liquid product containing the diol and a solid product containing the dicarboxylate can be typically obtained. The liquid product containing the diol and the solid product containing the dicarboxylate may be obtained as a mixture thereof, or may be obtained independently. It is preferred that the liquid product containing the diol and the solid product containing the dicarboxylate be obtained independently because the diol and the dicarboxylate are obtained more efficiently.

Any appropriate polyester-based resin may be adopted as the polyester-based resin to the extent that the effects of the present invention are not impaired as long as the polyester-based resin can produce a diol and a dicarboxylate by the decomposition method for a polyester-based resin according to the embodiment of the present invention. Examples of such polyester-based resin include: polyalkylene terephthalates, such as polyethylene terephthalate (PET), polypropylene terephthalate, polybutylene terephthalate, polycyclohexanedimethylene terephthalate, and polyhexylene terephthalate; polyethylene-2,6-naphthalenedicarboxylate (PEN); polybutylene-2,6-naphthalenedicarboxylate (PBN); polyethylene-1,2-bis(phenoxy)ethane-4,4'-dicarboxylate; polyethylene isophthalate/terephthalate; polybutylene isophthalate/terephthalate; polybutylene terephthalate/decanedicarboxylate; poly(ethylene terephthalate/cyclohexanedimethylene terephthalate); and polyethylene-4,4'-dicarboxylate/terephthalate. A typical example of the polyester-based resin is polyethylene terephthalate (PET).

A recycled polyester-based resin may be used as the polyester-based resin. The recycled polyester-based resin is obtained by, for example, collecting a used polyester-based resin film or a container such as a used waste polyester-based resin bottle, and subjecting the collected film or container to treatment such as purification. A typical example of the recycled polyester-based resin is recycled PET.

A virgin polyester-based resin may be used alone as the polyester-based resin, or a recycled polyester-based resin may be used alone. Alternatively, a blend of the virgin polyester-based resin and the recycled polyester-based resin may be used.

The diol obtained by the decomposition method for a polyester-based resin according to the embodiment of the present invention depends on the polyester-based resin to be used, and is, for example, an aliphatic diol having 2 to 20 carbon atoms. Specific examples thereof include ethylene glycol, propylene glycol, 1,4-butanediol, neopentyl glycol, 1,5-pentanediol, 1,6-hexanediol, decamethylene glycol, cyclohexanedimethanol, and cyclohexanediol. When PET is used as the polyester-based resin, the diol is, for example, ethylene glycol.

The dicarboxylate obtained by the decomposition method for a polyester-based resin according to the embodiment of the present invention depends on the polyester-based resin to be used, and examples thereof include a terephthalate, an isophthalate, an orthophthalate, a 2,6-naphthalenedicarboxylate, a 1,5-naphthalenedicarboxylate, an anthracenedicarboxylate, 4,4'-diphenyldicarboxylate, a 1,2-bis(phenoxy)ethane-4,4'-dicarboxylate, a sodium 5-sulfoisophthalate, an adipate, a sebacate, an azelate, a dodecanedioate, a 1,3-cyclohexanedicarboxylate, and a 1,4-cyclohexanedicarboxylate. When PET is used as the polyester-based resin, the dicarboxylate is, for example, a terephthalate.

Examples of a salt for forming the dicarboxylate include alkali metal salts, alkaline earth metal salts, and ammonium salts, and preferred examples thereof include: a salt of an alkali metal cation that may be incorporated into the solid alkali compound used in the decomposition method for a polyester-based resin according to the embodiment of the present invention; a salt of an alkaline earth metal cation that may be incorporated into the solid alkali compound used in the decomposition method for a polyester-based resin according to the embodiment of the present invention; and a salt of an ammonium cation that may be incorporated into the solid alkali compound used in the decomposition method for a polyester-based resin according to the embodiment of the present invention. Typical examples of the salt for forming the dicarboxylate include potassium salts and sodium salts.

The solid alkali compound is an alkali compound that is solid at normal temperature (typically 23°C) and normal pressure (typically under atmospheric pressure), and is an alkali compound that is solid at the time of its mixing with the polyester-based resin. Examples thereof include potassium hydroxide (KOH), sodium hydroxide (NaOH), and calcium hydroxide (Ca(OH)₂).

The total content ratio of the polyester-based resin and the solid alkali compound in the mixture containing the polyester-based resin and the solid alkali compound is, for example, from 80% by weight to 100% by weight, and may be from 90% by weight to 100% by weight, may be from 95% by weight to 100% by weight, may be from 98% by weight to 100% by weight, may be from 99% by weight to 100% by weight, or may be substantially 100% by weight.

The mixture containing the polyester-based resin and the solid alkali compound is preferably free of a solvent (e.g., an organic solvent or water). The content ratio of the solvent in the mixture containing the polyester-based resin and the solid alkali compound is, for example, from 0% by weight to 10% by weight, and may be from 0% by weight to 5% by weight, may be from 0% by weight to 3% by weight, may be from 0% by weight to 1% by weight, or may be substantially 0% by weight. The term "solvent" as used herein means a solvent used in combination with the polyester-based resin and the solid alkali compound, and does not encompass a solvent that may be incorporated in a trace amount into each of the polyester-based resin and the solid alkali compound, such as a solvent that may be inevitably incorporated during the production process or storage of the polyester-based resin or the solid alkali compound, or a solvent that may be incorporated into the polyester-based resin or the solid alkali compound serving as a product.

The mixture containing the polyester-based resin and the solid alkali compound may contain any appropriate other material (excluding a solvent) except the polyester-based resin and the solid alkali compound to the extent that the effects of the present invention are not impaired. The content ratio of the other material (excluding a solvent) in the mixture containing the polyester-based resin and the solid alkali compound is, for example, from 0% by weight to 10% by weight, and may be from 0% by weight to 5% by weight, may be from 0% by weight to 2% by weight, may be from 0% by weight to 1% by weight, or may be substantially 0% by weight.

The mixture containing the polyester-based resin and the solid alkali compound may be obtained by any appropriate method to the extent that the effects of the present invention are not impaired. The mixture containing the polyester-based resin and the solid alkali compound is typically obtained by mixing the polyester-based resin, the solid alkali compound, and as required, any other material. The polyester-based resin, the solid alkali compound, and as required, the other material may be mixed by any appropriate method to the extent that the effects of the present invention are not impaired. Such method may be a method including mixing the materials before their kneading (i.e., mixing the materials to prepare a mixture and then kneading the mixture), or a method including mixing the materials so that their kneading may be performed simultaneously with the mixing.

The polyester-based resin at the time of its mixing with the solid alkali compound may be a pulverized product. When the polyester-based resin is a pulverized product, its depolymerization can proceed favorably because its surface in contact with the solid alkali compound can increase. The pulverized product only needs to be produced through the pulverization of the polyester-based resin by any appropriate pulverization method to the extent that the effects of the present invention are not impaired. Such pulverization method is, for example, a pulverization method including using a pulverizer. Any appropriate size may be adopted as the size of the pulverized product to the extent that the effects of the present invention are not impaired. For example, the size of such pulverized product may be from 0.5 mmφ to 50 mmφ, may be from 0.5 mmφ to 30 mmφ, or may be from 0.5 mmφ to 20 mmφ in terms of the diameter of a screen for pulverization.

Any appropriate shape may be adopted as the shape of the solid alkali compound at the time of its mixing with the polyester-based resin to the extent that the effects of the present invention are not impaired. Examples of such shape include a granular shape and a powder shape. The solid alkali compound may be a pulverized product.

Any appropriate usage ratio may be adopted as a usage ratio between the polyester-based resin and the solid alkali compound to the extent that the effects of the present invention are not impaired. The usage ratio between the polyester-based resin and the solid alkali compound is, for example, 0.8 molar equivalent or more, and may be from 0.9 molar equivalent to 2.0 molar equivalents, may be from 0.95 molar equivalent to 1.8 molar equivalents, may be from 1.0 molar equivalent to 1.6 molar equivalents, may be from 1.0 molar equivalent to 1.5 molar equivalents, may be from 1.0 molar equivalent to 1.4 molar equivalents, may be from 1.0 molar equivalent to 1.3 molar equivalents, may be from 1.0 molar equivalent to 1.2 molar equivalents, or may be from 1.0 molar equivalent to 1.1 molar equivalents in terms of the molar equivalent of the solid alkali compound relative to 1 molar equivalent of the polyester-based resin because the effects of the present invention can be exhibited to a larger extent. When the molar equivalent of the solid alkali compound relative to 1 molar equivalent of the polyester-based resin is so low as to deviate from the above-mentioned ranges, the yield of a target product may reduce because the depolymerization of the polyester-based resin does not proceed sufficiently. When the molar equivalent of the solid alkali compound relative to 1 molar equivalent of the polyester-based resin is so high as to deviate from the above-mentioned ranges, the yield of the target product may reduce because a side reaction occurs, or the work burden of post-treatment may increase because a large amount of the alkali compound remains.

The kneading of the mixture containing the polyester-based resin and the solid alkali compound may be performed with any appropriate apparatus to the extent that the effects of the present invention are not impaired. Such apparatus is a decomposition apparatus that decomposes a polyester-based resin to produce a diol and a dicarboxylate, and is also a decomposition apparatus for a polyester-based resin according to an embodiment of the present invention.

That is, the decomposition apparatus for a polyester-based resin according to an embodiment of the present invention is a decomposition apparatus configured to decompose a polyester-based resin to produce a diol and a dicarboxylate, and includes a kneading portion configured to knead a mixture containing the polyester-based resin and a solid alkali compound.

The decomposition apparatus may further include: a first discharge portion configured to discharge a liquid product containing the diol; and a second discharge portion configured to discharge a solid product containing the dicarboxylate because the effects of the present invention can be exhibited to a larger extent. When the decomposition apparatus independently including the first discharge portion configured to discharge the liquid product containing the diol and the second discharge portion configured to discharge the solid product containing the dicarboxylate is prepared as described above, the decomposition of the polyester-based resin can proceed efficiently, and hence the diol and the dicarboxylate can be produced efficiently.

Examples of the decomposition apparatus include a twin-screw continuous kneader, a twin-screw continuous reactor, and a batch type reaction vessel.

The decomposition method for a polyester-based resin according to the embodiment of the present invention is described more specifically by taking a case in which a twin-screw extruder is applied as the decomposition apparatus as an example. The twin-screw extruder typically includes a raw material feed portion, a kneading portion, a vent portion, and a discharge portion. The vent portion corresponds to the above-mentioned first discharge portion, and the discharge portion corresponds to the above-mentioned second discharge portion. The kneading portion may include a plurality of kneading/reaction sections in each of which temperature adjustment is possible. When the twin-screw extruder is applied as the decomposition apparatus used in the decomposition method for a polyester-based resin according to the embodiment of the present invention,
(1) the mixture containing the polyester-based resin and the solid alkali compound (prepared in advance) is, or the polyester-based resin, the solid alkali compound, and as required, any other material are, fed from the raw material feed portion,
(2) the mixture containing the polyester-based resin and the solid alkali compound is kneaded in the kneading portion,
(3) the produced liquid product containing the diol is discharged from the vent portion (corresponding to the first discharge portion), and
(4) the produced solid product containing the dicarboxylate is discharged from the discharge portion (corresponding to the second discharge portion).

Any appropriate temperature may be adopted as the temperature of the kneading of the mixture containing the polyester-based resin and the solid alkali compound to the extent that the effects of the present invention are not impaired. The temperature of the kneading of the mixture containing the polyester-based resin and the solid alkali compound is, for example, 50°C or more and less than 400°C, and may be 70°C or more and less than 350°C, may be 80°C or more and less than 320°C, may be 90°C or more and less than 300°C, may be 100°C or more and less than 280°C, may be 120°C or more and less than 270°C, may be 150°C or more and less than 270°C, may be 180°C or more and less than 270°C, or may be 200°C or more and less than 270°C because the effects of the present invention can be exhibited to a larger extent. When the temperature of the kneading of the mixture containing the polyester-based resin and the solid alkali compound is so low as to deviate from the above-mentioned ranges, the yield of the target product may reduce because the depolymerization of the polyester-based resin does not proceed sufficiently. When the temperature of the kneading of the mixture containing the polyester-based resin and the solid alkali compound is so high as to deviate from the above-mentioned ranges, the yield of the target product may reduce because the solid alkali compound melts or a side reaction occurs.

When the decomposition apparatus includes a kneading portion including a plurality of kneading/reaction sections in each of which temperature adjustment is possible, the temperatures of kneading in all the kneading/reaction sections preferably fall within the above-mentioned ranges of the temperature of the kneading of the mixture containing the polyester-based resin and the solid alkali compound.

In the decomposition apparatus, any appropriate pressure may be adopted as the pressure of the kneading portion to the extent that the effects of the present invention are not impaired. The pressure of the kneading portion is preferably adjusted from reduced pressure to normal pressure. Specifically, when the atmospheric pressure is 0 MPa in gauge pressure indication, the pressure of the kneading portion is, for example, from -0.1 MPa to normal pressure, and may be from -0.1 MPa to -0.001 MPa, may be from -0.1 MPa to -0.005 MPa, or may be from -0.1 MPa to -0.01 MPa. In particular, the pressure is preferably under reduced pressure, and for example, may be from -0.1 MPa to -0.001 MPa, may be from -0.1 MPa to -0.005 MPa, or may be from -0.1 MPa to -0.01 MPa from the viewpoint of reaction efficiency, from the viewpoint of suppressing the scattering of the solid alkali compound, and when a solid alkali compound that is liable to deliquesce is used, from the viewpoint of suppressing the deliquescence of the solid alkali compound.

When the decomposition apparatus includes the kneading portion including the plurality of kneading/reaction sections in each of which temperature adjustment is possible, the pressures in all the kneading/reaction sections preferably fall within the above-mentioned pressure ranges.

When the decomposition apparatus is a continuous type apparatus (e.g., a twin-screw extruder), a residence time in the decomposition apparatus is, for example, less than 1 hour, and may be 0.1 minute or more and less than 45 minutes, may be 0.1 minute or more and less than 30 minutes, may be 0.1 minute or more and less than 15 minutes, may be 0.1 minute or more and less than 10 minutes, may be 0.2 minute or more and less than 5 minutes, may be 0.3 minute or more and less than 3 minutes, may be 0.4 minute or more and less than 2 minutes, or may be 0.5 minute or more and less than 1.5 minutes. According to the decomposition method for a polyester-based resin according to the embodiment of the present invention, the diol and the dicarboxylate can be produced from the polyester-based resin in such short time period.

When the decomposition apparatus is a batch type apparatus (e.g., a batch type reaction vessel), a reaction time in the decomposition apparatus is, for example, less than 2 hours, and may be 0.1 minute or more and less than 1.5 hours, may be 0.1 minute or more and less than 1 hour, may be 0.1 minute or more and less than 45 minutes, may be 0.1 minute or more and less than 30 minutes, may be 0.1 minute or more and less than 15 minutes, may be 0.1 minute or more and less than 10 minutes, may be 0.2 minute or more and less than 5 minutes, may be 0.3 minute or more and less than 3 minutes, may be 0.4 minute or more and less than 2 minutes, or may be 0.5 minute or more and less than 1.5 minutes. According to the decomposition method for a polyester-based resin according to the embodiment of the present invention, the diol and the dicarboxylate can be produced from the polyester-based resin in such short time period.

When the decomposition apparatus is a continuous type apparatus, any appropriate supply rate may be adopted as the supply rate of the raw material (the mixture containing the polyester-based resin and the solid alkali compound (prepared in advance), or the polyester-based resin, the solid alkali compound, and as required, any other material) to the extent that the effects of the present invention are not impaired. Such supply rate is, for example, from 0.01 kg/hr to 100 kg/hr, and may be from 0.05 kg/hr to 50 kg/hr, may be from 0.1 kg/hr to 20 kg/hr, may be from 0.5 kg/hr to 10 kg/hr, or may be from 1.0 kg/hr to 5.0 kg/hr.

The diol may be recovered from the liquid product containing the diol, which may be obtained by the decomposition method for a polyester-based resin according to the embodiment of the present invention, by using any appropriate method.

The dicarboxylate may be recovered from the solid product containing the dicarboxylate, which may be obtained by the decomposition method for a polyester-based resin according to the embodiment of the present invention, by using any appropriate method.

A dicarboxylic acid may be obtained from the solid product containing the dicarboxylate, which may be obtained by the decomposition method for a polyester-based resin according to the embodiment of the present invention, by using any appropriate method. Such method is, for example, neutralization crystallization, and specifically, a crystal of the dicarboxylic acid is precipitated by turning the solid product containing the dicarboxylate into an aqueous solution and adding an acid to the aqueous solution. In one embodiment, when potassium hydroxide (KOH) is used as the solid alkali compound, the solid product contains a potassium salt of the dicarboxylic acid and unreacted solid potassium hydroxide. Water is added to the solid product to provide an aqueous solution, and an acid (e.g., acetic acid) is added to the aqueous solution to perform neutralization crystallization. Thus, a mixture of the crystal of the dicarboxylic acid, water, and potassium acetate is obtained. The mixture is subjected to solid-liquid separation, and the resultant solid is washed and dried as required. Thus, the dicarboxylic acid may be obtained.

### EXAMPLES

The present invention is specifically described below by way of Examples. However, the present invention is by no means limited to these Examples. Test and evaluation methods in Examples and the like are as described below.

### <<Calculation of Dicarboxylic Acid Recovery Rate>>

The amount of a dicarboxylate theoretically produced by a depolymerization reaction was calculated from the amounts of a polyester-based resin and a solid alkali compound fed into a decomposition apparatus, and the amount W1 of a dicarboxylic acid theoretically obtained by subjecting the dicarboxylate to neutralization crystallization was calculated from the amount of the dicarboxylate.

The amount of the dicarboxylic acid actually obtained was defined as W2, and a dicarboxylic acid recovery rate was determined by the following equation. $Dicarboxylic acid recovery rate \left(%\right) = \left(W2/W1\right) \times 100 \left(%\right)$

The amounts W1 and W2 only need to be weights per predetermined time (e.g., g/30 seconds as a unit) in the case of a continuous type decomposition apparatus, and only need to be obtained weights (e.g., "g" as a unit) in the case of a batch type decomposition apparatus.

### [Example 1]

A PET film (manufactured by Mitsubishi Chemical Corporation, Diafoil T100, thickness=38 µm) was pulverized with a pulverizer (manufactured by Yoshikoh Co., Ltd., RC250) including a screen of 10 mmφ to provide a pulverized sample.

A twin-screw continuous kneader/reactor (manufactured by Kurimoto, Ltd., S2KRC Kneader) was used as a decomposition apparatus used for decomposition.

The pulverized sample and solid KOH were mixed so that the stoichiometric ratio "pulverized sample:KOH" (molar equivalent ratio) became 1:1. After that, the mixture was fed into the raw material feed portion of the S2KRC Kneader at 2 kg/hr.

To suppress the scattering of KOH and to efficiently perform a decomposition reaction (depolymerization reaction) in the system, a pressure in the entire reaction system was set to a slightly reduced pressure (-0.01 MPa), and the inside thereof was purged with N₂ to suppress the deliquescence of KOH. The temperatures of the S2KRC Kneader were set to 70°C, 250°C, and 241°C for three kneading/reaction sections from the raw material feed portion side of the kneader toward the discharge portion side thereof.

An estimated residence time in the S2KRC Kneader was estimated to be about 1 minute.

Ethylene glycol produced by the depolymerization reaction was recovered under reduced pressure as a liquid product from the vent portion of the kneader.

Potassium terephthalate (TPK) produced by the depolymerization reaction was recovered as a solid product from the discharge portion of the kneader. The resultant solid product was in a powder form.

The recovered powder was dissolved in water to provide an aqueous solution, and the solution was subjected to neutralization crystallization with acetic acid and to solid-liquid separation. The resultant solid was washed with water and then dried at 130°C for 30 minutes to provide solid terephthalic acid (TPA).

The results are shown in Table 1 and Table 2.

### [Example 2]

A PET film (manufactured by Mitsubishi Chemical Corporation, Diafoil T100, thickness=38 µm) was pulverized with a pulverizer (manufactured by Yoshikoh Co., Ltd., RC250) including a screen of 3 mmφ to provide a pulverized sample.

A twin-screw continuous kneader/reactor (manufactured by Kurimoto, Ltd., U-trough KRC Kneader) was used as a decomposition apparatus used for decomposition.

The pulverized sample and solid KOH were mixed so that the stoichiometric ratio "pulverized sample:KOH" (molar equivalent ratio) became 1:1. After that, the mixture was fed into the raw material feed portion of the U-trough KRC Kneader at 2 kg/hr.

A pressure in the entire reaction system was set to normal pressure. The temperatures of the U-trough KRC Kneader were set to 150°C, 150°C, and 150°C for three kneading/reaction sections from the raw material feed portion side of the kneader toward the discharge portion side thereof.

An estimated residence time in the U-trough KRC Kneader was estimated to be about 1 minute.

Ethylene glycol produced by the depolymerization reaction was recovered under reduced pressure as a liquid product from the vent portion of the kneader.

Potassium terephthalate (TPK) produced by the depolymerization reaction was recovered as a solid product from the discharge portion of the kneader. The resultant solid product was in a powder form.

The recovered powder was dissolved in water to provide an aqueous solution, and the solution was subjected to neutralization crystallization with acetic acid and to solid-liquid separation. The resultant solid was washed with water and then dried at 130°C for 30 minutes to provide solid terephthalic acid (TPA).

The results are shown in Table 1 and Table 2.

### [Example 3]

A PET film (manufactured by Mitsubishi Chemical Corporation, Diafoil T100, thickness=38 µm) was pulverized with a pulverizer (manufactured by Yoshikoh Co., Ltd., RC250) including a screen of 3 mmφ to provide a pulverized sample.

A twin-screw continuous kneader/reactor (manufactured by Kurimoto, Ltd., U-trough KRC Kneader) was used as a decomposition apparatus used for decomposition.

The pulverized sample and solid KOH were mixed so that the stoichiometric ratio "pulverized sample:KOH" (molar equivalent ratio) became 1:1.5. After that, the mixture was fed into the raw material feed portion of the U-trough KRC Kneader at 2 kg/hr.

To suppress the scattering of KOH and to efficiently perform a decomposition reaction (depolymerization reaction) in the system, a pressure in the entire reaction system was set to a slightly reduced pressure (-0.01 MPa), and the inside thereof was purged with N₂ to suppress the deliquescence of KOH. The temperatures of the U-trough KRC Kneader were set to 250°C, 250°C, and 250°C for three kneading/reaction sections from the raw material feed portion side of the kneader toward the discharge portion side thereof.

An estimated residence time in the U-trough KRC Kneader was estimated to be about 1 minute.

Ethylene glycol produced by the depolymerization reaction was recovered under reduced pressure as a liquid product from the vent portion of the kneader.

Potassium terephthalate (TPK) produced by the depolymerization reaction was recovered as a solid product from the discharge portion of the kneader. The resultant solid product was in a powder form.

The recovered powder was dissolved in water to provide an aqueous solution, and the solution was subjected to neutralization crystallization with acetic acid and to solid-liquid separation. The resultant solid was washed with water and then dried at 130°C for 30 minutes to provide solid terephthalic acid (TPA).

The results are shown in Table 1 and Table 2.

### [Example 4]

A PET film (manufactured by Mitsubishi Chemical Corporation, Diafoil T100, thickness=38 µm) was pulverized with a pulverizer (manufactured by Yoshikoh Co., Ltd., RC250) including a screen of 3 mmφ to provide a pulverized sample.

The pulverized sample and solid KOH were fed into a 1 L separable flask so that the stoichiometric ratio "pulverized sample:KOH" (molar equivalent ratio) became 1:1.5. The mixture was stirred with a three-one motor while being heated with a heater at 150°C under reduced pressure (-0.1 MPa).

Produced ethylene glycol was recovered with a trap.

The depolymerization reaction of the sample proceeded dramatically within 1 minute from the start of the stirring. Potassium terephthalate (TPK) produced by the depolymerization reaction was recovered as a solid product from the separable flask. The resultant solid product was in a powder form.

The recovered powder was dissolved in water to provide an aqueous solution, and the solution was subjected to neutralization crystallization with acetic acid and to solid-liquid separation. The resultant solid was washed with water and then dried at 130°C for 30 minutes to provide solid terephthalic acid (TPA).

The results are shown in Table 1 and Table 2.

### [Example 5]

A PET film (manufactured by Mitsubishi Chemical Corporation, Diafoil T100, thickness=38 µm) was pulverized with a pulverizer (manufactured by Yoshikoh Co., Ltd., RC250) including a screen of 3 mmφ to provide a pulverized sample.

The pulverized sample and solid KOH were fed into a 20 L batch type polymerization vessel so that the stoichiometric ratio "pulverized sample:KOH" (molar equivalent ratio) became 1:1. The mixture was stirred with a three-one motor while being heated with a heater at 235°C under reduced pressure (-0.1 MPa).

Produced ethylene glycol was recovered with a trap.

After the stirring for 1 hour, potassium terephthalate (TPK) produced by the depolymerization reaction of the sample was recovered as a solid product from the polymerization vessel. The resultant solid product was in a powder form.

The recovered powder was dissolved in water to provide an aqueous solution, and the solution was subjected to neutralization crystallization with acetic acid and to solid-liquid separation. The resultant solid was washed with water and then dried at 130°C for 30 minutes to provide solid terephthalic acid (TPA).

The results are shown in Table 1 and Table 2.

**Table 1**

| | Polyester-based resin | Solid alkali compound | Polyester-based resin:solid alkali compound (molar equivalent ratio) | Decomposition apparatus | Temperature | Pressure |
|---|---|---|---|---|---|---|
| Example 1 | PET | KOH | 1:1 | S2KRC Kneader | 70°C/250°C/241°C | -0.01 MPa |
| Example 2 | PET | KOH | 1:1 | U-trough KRC Kneader | 150°C/150°C/150°C | Normal pressure |
| Example 3 | PET | KOH | 1:1.5 | U-trough KRC Kneader | 250°C/250°C/250°C | -0.01 MPa |
| Example 4 | PET | KOH | 1:1.5 | Separable flask | 150°C | -0.1 MPa |
| Example 5 | PET | KOH | 1:1 | Polvmerization vessel | 235°C | -0.1 MPa |

**Table 2**

| | Actual supply amount | | Theoretical value of discharge amount | | | Actual recovery amount | Dicarboxylic acid recovery rate (%) |
|---|---|---|---|---|---|---|---|
| | PET | KOH | TPK | KOH | TPA obtained from TPK | TPA | |
| Example 1 | 19.9 (g/30 sec) | 11.5 (g/30 sec) | 24.89 (g/30 sec) | 0.0 (g/30 sec) | 17.0 (g/30 sec) | 13.5 (g/30 sec) | 79.0 |
| Example 2 | 19.9 (g/30 sec) | 11.5 (g/30 sec) | 24.89 (g/30 sec) | 0.0 (g/30 sec) | 17.0 (g/30 sec) | 15.7 (g/30 sec) | 92.4 |
| Example 3 | 16.5 (g/30 sec) | 14.3 (g/30 sec) | 20.69 (g/30 sec) | 4.8 (g/30 sec) | 14.1 (g/30 sec) | 9.9 (g/30 sec) | 70.0 |
| Example 4 | 69.7 (g) | 60.4 (g) | 87.0 (g) | 20.1 (g) | 59.6 (g) | 48.7 (g) | 81.7 |
| Example 5 | 2000 (g) | 1156 (g) | 2496 (g) | 0.0 (g) | 1711 (g) | 1394 (g) | 81.5 |

The decomposition method and decomposition apparatus for a polyester-based resin according to the embodiments of the present invention can each be suitably utilized for the chemical recycling of a polyester-based resin.

## Claims

1. A decomposition method for a polyester-based resin, comprising kneading a mixture containing a polyester-based resin and a solid alkali compound to produce a diol and a dicarboxylate.

2. The decomposition method for a polyester-based resin according to claim 1, wherein a molar equivalent of the solid alkali compound relative to 1 molar equivalent of the polyester-based resin is 0.8 molar equivalent or more.

3. The decomposition method for a polyester-based resin according to claim 2, wherein the molar equivalent of the solid alkali compound relative to 1 molar equivalent of the polyester-based resin is from 1.0 molar equivalent to 1.5 molar equivalents.

4. The decomposition method for a polyester-based resin according to any one of claims 1 to 3, wherein a temperature of the kneading is 50°C or more and less than 400°C.

5. The decomposition method for a polyester-based resin according to claim 4, wherein the temperature of the kneading is 120°C or more and less than 270°C.

6. The decomposition method for a polyester-based resin according to any one of claims 1 to 5, wherein a liquid product containing the diol and a solid product containing the dicarboxylate are obtained by the kneading.

7. The decomposition method for a polyester-based resin according to any one of claims 1 to 6, wherein the dicarboxylate is subjected to neutralization crystallization to produce a dicarboxylic acid.

8. A decomposition apparatus for a polyester-based resin, which is configured to decompose a polyester-based resin to produce a diol and a dicarboxylate, the decomposition apparatus comprising a kneading portion configured to knead a mixture containing the polyester-based resin and a solid alkali compound.

9. The decomposition apparatus for a polyester-based resin according to claim 8, further comprising:
a first discharge portion configured to discharge a liquid product containing the diol; and
a second discharge portion configured to discharge a solid product containing the dicarboxylate.
